Europäisches Patentamt

European Patent Office

Office européen des brevets

(11) Publication number: 0 386 979
A2

# EUROPEAN PATENT APPLICATION

(21) Application number: 90302322.4

(22) Date of filing: 05.03.90

(51) Int. Cl.⁵: C12N 15/16, C12P 21/02, A61K 37/24, C12Q 1/68, C07K 13/00

(30) Priority: 06.03.89 US 319585

(43) Date of publication of application:
12.09.90 Bulletin 90/37

(84) Designated Contracting States:
AT BE CH DE DK ES FR GB GR IT LI LU NL SE

(71) Applicant: GENENTECH, INC.
460 Point San Bruno Boulevard
South San Francisco California 94080(US)

(72) Inventor: Wood, William I.
1400 Terrytown Street
San Mateo, California 94402(US)
Inventor: Colosi, Peter C.
2115 1/1 Ashby Avenue
Berkeley, California 94705(US)

(74) Representative: Armitage, Ian Michael et al
MEWBURN ELLIS 2 Cursitor Street
London EC4A 1BQ(GB)

(54) DNA encoding ovine-active, growth-hormone-receptor-binding, lactogenic protein.

(57) A nucleic-acid molecule encoding a biologically active protein that binds growth hormone receptor can be isolated and expressed to obtain the protein for use in treating sheep and goats, thereby to induce lactogenesis, an increase in muscle, an increase in mammary gland mass and/or a decrease in fat content.

EP 0 386 979 A2

## DNA ENCODING OVINE-ACTIVE, GROWTH-HORMONE-RECEPTOR-BINDING, LACTOGENIC PROTEIN

### Background of the Invention

The present invention relates to means for producing a protein that displays lactogenic activity and binds growth-hormone (GH) receptor, and to the use of the protein to enhance muscle or mammary-gland mass or to decrease fat content in ruminant mammals.

Certain protein preparations derived from the placentas of ruminants have been shown to be active in prolactin bioassays, and the components presumed responsible for this activity have been termed "lactogens." Further characterization of these lactogenic factors has been hampered by technical problems, however. For example, reports on efforts to purify placental lactogens extracted from sheep and goats ("ovine placental lactogens") have appeared periodically in the literature over more than a decade, but no amino-acid sequence for an ovine placental lactogen protein (oPL) has yet been published.

A wide range of isoelectric-point values has been associated with ovine-placental lactogenic activity, indicating a compositional diversity in the protein preparations under study. Consistent with this indication is the finding of more than one protein form comprising a purportedly "homogeneous" lactogen preparation. Chan et al., FEBS Lett. 199(2): 259-64 (1986). Also, low yields have hindered detailed study of these proteins (see, e.g., Chan et al., supra, at page 259).

As a consequence, relatively little is known about the characteristics, and particularly the physiological properties, of placental lactogen factors. Nevertheless, ovine placental lactogens are of potential commercial interest because they are among the few substances, other than human growth hormone (hGH), known to bind human GH receptor. See, e.g., Chan et al., Endocrinology 98: 65-76 (1976).

While this finding might be taken to suggest that ovine placental lactogens, at least in adults, might bind an ovine GH receptor and, thereby, mimic the growth-affecting properties of endogenous growth hormone, there is experimental evidence indicating that growth factors other than an ovine placental lactogen are primary growth determinants. Compare Schuler in REGULATION OF GROWTH AND LACTATION IN ANIMALS 107-113 (Endocrine Society 1985) with Thordarson et al., J. Endocr. 113: 277-83 (1987). In addition, it has not been determined whether ovine or bovine placental lactogens stimulate milk synthesis in goat, sheep or cow cell tissue, and anti-ovine-placental-lactogen serum does not cross react with ovine growth hormone. Reddy & Watkins, J. Endocrinol. 78: 59, 68-69 (1978); Martal & Djiane, Biochem. Biophys. Res. Comm. 65(2): 770, 776 (1975). Accordingly, there is an expectation that these lactogens are not biologically active in a manner similar to that of endogenous lactogens or growth hormone, and consequently would not be useful as a GH mimic or as a milk production stimulator. Schuler, supra, at page 109.

The aforementioned findings are inconsistent with the stimulation of milk production, via administration to cows of a bovine placental lactogen factor, disclosed in U.S. patent No. 4,767,711. This patent also discloses the cloning from DNA obtained from cow placental cotyledons of a DNA encoding a lactogenic factor. But a lack of information as to the degree of structural homology, if any, between a bovine placental lactogens and placental lactogens of other mammals has rendered unpredictable the designing of usable hybridization probes from extant data on bovine material.

### Summary of the Invention

It is therefore an object of the present invention to provide a nucleic-acid molecule encoding a lactogenic protein that, while bearing little similarity to human growth hormone and only some similarity to bovine placental lactogen, binds human as well as ovine GH receptors.

It is also an object of the present invention to provide a method for using the aforementioned protein to enhance specific, commercially significant developmental and lactogenic characteristics of ruminant mammals without a concomitant, undesirable increase in fatty-tissue mass.

In accomplishing these objects, there has been provided, in accordance with one aspect of the present invention, an isolated nucleic-acid molecule comprised of a nucleotide sequence encoding a protein that binds a mammalian growth hormone receptor, wherein the protein is characterized by an amino-acid-sequence identity of about 26% relative to human growth hormone and about 67% relative to bovine placental lactogen.

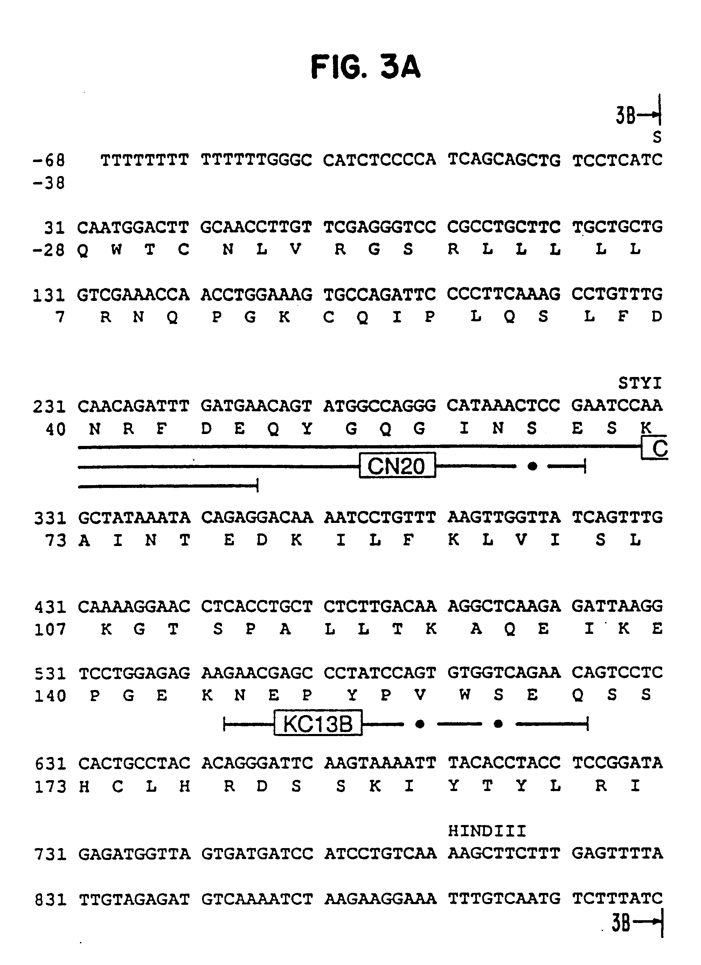

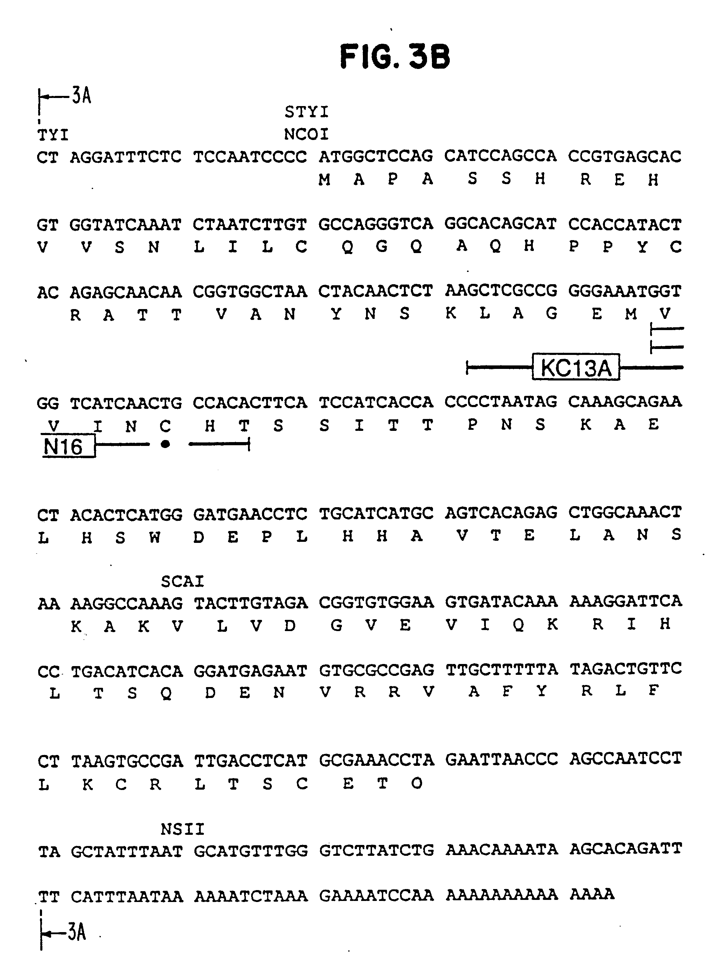

In accordance with another aspect of the present invention, a composition has been provided that comprises (a) homogeneous protein in pure form having the aforementioned functional and structural characteristics and (b) a physiologically-compatible carrier. In a preferred embodiment, component (a) of the composition consists essentially of an amino-acid sequence of residue +1 (gln) through residue +198 (thr) in Figure 3 below.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description. Unless otherwise indicated, the literature cited below is hereby incorporated by reference.

## Brief Description of the Drawings

Figures 1A and 1B are chromatograms depicting data from reverse-phase high performance liquid chromatography (RP-HPLC) that characterize, respectively, cyanogen bromide- and lysine-C endopeptidase-digested fragments of an ovine placental lactogen protein.

Figure 2 presents the amino-acid sequences of two polypeptides (KC13a and CN16) obtained via digestion of an ovine placental lactogen protein. Shown are two synthetic oligonucleotide probes, designated "oopl.1" and "oopl.2," the respective sequences of which are based on the KC13a and CN16 sequences. Also shown is the nucleotide sequence for an isolated cDNA molecule designated "λopl.7." (Asterisks indicate matching nucleotides between the cDNA and the probes.)

Figure 3 depicts the full nucleotide and encoded amino-acid sequences for cDNAs selected by hybridization to a synthetic oligonucleotide probe prepared on the basis of a 29 amino-acid sequence determined from KC13 and CN16. The locations of four peptides from Table 1 below are indicated.

Figure 4 shows an autoradiograph from a northern blot which evidences the detection of ovine placental RNA corresponding to the full-length cDNA insert from clone λopl.7.

Figure 5 is a line drawing that provides a schematic depiction of the cDNA insert of Figure 3 and corresponding restriction sites, which cDNA predicts a mature protein of 198 amino acids, including six cysteine residues. A hydrophobicity plot is also provided.

Figure 6 depicts an amino acid-sequence alignment of an ovine-active, GH-receptor-binding protein with placental lactogen proteins from a number of species and, in addition, with human growth hormone, ovine growth hormone and ovine prolactin. Boxed residues match those of the GH-receptor-binding protein.

Figure 7 is a schematic representation of the construction of vector pRK5.opll.1 from three DNA segments.

Figure 8 is a schematic representation of the construction of vector pTrp.opl.

## Detailed Description of the Preferred Embodiments

Notwithstanding previous indications which militated against an endogenous biological activity for an ovine-active protein with both lactogenic and GH-like properties, it has been discovered that a lactogenic protein is produced in ovine placental tissue that mimics GH endogenously and that is coded for by a clonable DNA segment.

In this description, "clonable" denotes a nucleotide sequence, typically coding for a desired amino-acid seqence, that can be isolated from its normal setting, amplified, and expressed heterologously in usable quantities.

As a consequence, the present invention permits the production of a lactogenic protein distinguished by sequence homologies, in addition to advantageous physiological properties, that could not have been predicted from extant information on lactogenic factors. In particular, a nucleic-acid molecule within the present invention can be used to produce a lactogenic protein that binds GH receptor even though the protein is substantially nonhomologous (only 26% homology) to hGH and dissimilar (only 67% homology) to bovine placental lactogen, which binds GH receptor.

The amino acids which comprise sequences set out in the present description are designated, respectively, by the following single-letter symbols:

| Alanine (ala) | A | Leucine (leu) | L |
|---|---|---|---|
| Arginine (arg) | R | Lysine (lys) | K |
| Asparagine (asn) | N | Methionine (met) | M |
| Aspartic Acid (asp) | D | Phenylalanine (phe) | F |
| Cysteine (cys) | C | Proline (pro) | P |
| Glutamine (gln) | Q | Serine (ser) | S |
| Glutamic Acid (glu) | E | Threonine (thr) | T |
| Glycine (gly) | G | Tryptophan (trp) | W |
| Histidine (his) | H | Tyrosine (tyr) | Y |
| Isoleucine (ile) | I | Valine (val) | V |

To obtain DNA within the present invention, a oligonucleotide probe is designed, based on a protein that is known to bind GH receptor, by digesting the protein into one or more fragments of appropriate length for sequencing, generally about 30 amino-acid residues. Suitable cloning strategies and procedures are summarized, for example, by Frischauf et al., Nucleic Acid Res. 8: 5541 (1980). The protein fragments thus obtained are separated by RP-HPLC and sequenced. The sequences thus determined are then converted to the corresponding, amino-acid-encoding nucleotide sequence. Based on this nucleotide sequence, a labeled oligonucleotide probe can be synthesized chemically that will bind to a portion of a putative nucleotide sequence that encodes a GH-receptor-binding protein.

For RP-HPLC in accordance with the present invention, a C4-reverse phase column is preferably used. Alternatively, C3 or C1 columns can be employed. For developing the column, a solvent system is preferred that includes trifluoracetic acid (TFA) or a 1-propanol gradient.

The GH-receptor-binding protein that is digested for sequencing, pursuant to the present invention, is preferably an oPL protein, which can be obtained from ovine placenta by a procedure comprising the successive steps of homogenation, centrifugation, ammonium sulfate precipitation, dialysis, diethylaminoethyl (DEAE) cellulose anion exchange chromatography, an optional first gel filtration, cation exchange chromatography and second, desalting gel filtration. These steps follow the general method developed by Chan et al. (1976), supra, with certain modifications described below. The protein purification is preferably monitored at each step by means of a GH-receptor-binding assay, as described in greater detail below.

It has been discovered that fragments suitable for design of a probe can be obtained by digesting an oPL protein, purified as described in greater detail below, with cyanogen bromide or with lysine-C endopeptidase, followed by RP-HPLC (see below). These digestion agents were found to display adequate cleavage specificity and, at the same time, to provide fragments of a size suitable to produce oligonucleotide probes.

The design of a suitable oligonucleotide probe for use according to the present invention thus involves the isolation of peptide fragments from a protein, such as the aforementioned oPL protein, that is determined to bind a mammalian GH receptor, preferably an ovine or a human GH receptor. Sequence data derived from the fragments direct synthesis of oligonucleotide probes which, in turn, are used in screening a library of cDNA clones generated from placental mRNA obtained by conventional methods utilizing a reverse transcriptase. See, e.g., MANIATIS ET AL., MOLECULAR CLONING: A LABORATORY MANUAL, 261-401 (Cold Spring Harbour Laboratory 1982). In particular, a cDNA library suitable for this purpose can be generated from ovine placental mRNA. Alternatively, a probe can be tested in screening ovine genomic clones.

When a probe is identified that hybridizes to DNA comprising such a cDNA or genomic library under conditions of suitable stringency, see Wood, Methods Enzymol. 152: 443-47 (1987), the DNA recognized by the probe is isolated and incorporated into a suitable bacterial or viral expression vector and expressed in a bacterial or mammalian expression system. See MANIATIS ET AL., 403-33, and Okayama & Berg, Molecular & Cellular Biol. 2: 161-170 (1982).

Suitable for this purpose is an E. coli expression system as described, for example, by Leung et al., Bio/Technology 2: 458-64 (1984). Alternatively, nucleic-acid of the present invention can be incorporated into a mammalian expression vector, constructed to include, in addition to nucleic-acid encoding a GH-receptor-binding protein as described above, one or more selectable genes, a promotor that induces expression of the DNA to the extent of producing extractable amounts of protein, and restriction sites that allow for incorporation of these elements. In a preferred embodiment, the above-mentioned mammalian expression vector is pRK5, as disclosed in Suva et al., Science 237:893-6 (1987), the contents of which are hereby incorporated by reference.

An expression vector containing DNA within the present invention can be transfected, for example, into a cultured human or monkey kidney cell line. In a preferred embodiment, COS-7 monkey kidney cells or 293 human kidney cells are used for this purpose.

Expression of the desired protein can be assayed by means of a conventional in vitro GH-receptor-binding assay. For example, the culture medium of the transformed cells can be tested for human-GH-receptor-binding protein by performing a binding assay with a suitable source of GH receptor that includes cell membranes known to contain GH receptors. Binding is measured as the displacement of $^{125}$I-labelled human GH by culture supernatants containing the expressed protein, pursuant to the methodology, for example, of Spencer et al., J. Biol. Chem. 263: 7862-67 (1988). As a source of GH receptor, the GH-receptor-binding assay can employ mouse or rabbit liver membranes or human IM-9 cell membranes, or recombinant DNA-expressed rabbit or human GH receptor.

Expression of DNA obtained in above-described manner provides a source, in accordance with the present invention, for homogeneous protein (i.e., a preparation containing protein characterized by the same biological activity) that can be administered, preferably in pure form with a suitable carrier, to sheep, goats, cows or other test animals, to ascertain optimal pharmacological concentrations for producing GH-mimicking effects by the protein. These GH-mimicking effects include increased muscle and mammary-gland mass, and decreased fat content. In particular, a protein obtained via a probe derived from the oPL protein described above displays these physiological properties, as does a protein with the aforementioned, mature amino-acid sequence. Such protein can be used, pursuant to the present invention, to enhance growth and mammary gland size, thereby increasing, respectively, meat and milk production, and to decrease fat content of ruminants, especially sheep and goats. A lactogenic protein encoded by DNA within the present invention can also be used to increase milk production directly. In these contexts, the protein can be administered via the routes conventionally employed in administering growth hormone, such as by means of subcutaneous or intramuscular injection, in combination with a physiologically-compatible carrier such as physiological saline, or by parenteral slow-release from a suitable implant.

The present invention is further described below by reference to the following, illustrative examples.

EXAMPLE 1. Cloning and Expression of an Ovine Placental cDNA Expressing a Protein that Binds Human Growth Hormone Receptor.

OBTAINING SEQUENCEABLE oPL:

To the end of designing probes as described above, placental cotyledons are obtained surgically from pregnant ewes (110- to 120-days pregnant), and the fetal component is separated from the maternal tissue. For the extraction, 1.2 kg of the fetal tissue are homogenized, for example, with a Polytron PT-10 (Brinkmann) homogenizer at maximum speed for about 30 to 60 seconds, in 100 mM ammonium bicarbonate buffer adjusted to pH 9.5 with 1 N ammonium hydroxide (5:1 ratio (v/w) of buffer to tissue). The homogenate is then stirred overnight and centrifuged at 30,000 X g for 30 minutes, and the resulting pellet is discarded.

An ammonium sulfate precipitation can be carried out by adding ammonium sulfate slowly to the supernatant which is obtained from the above-mentioned centrifugation, resulting in a 30% saturated solution. The precipitate is allowed to settle overnight, and then is discarded. To the supernatant, enough additional ammonium sulfate is added to achieve a 75% saturated solution. The solution is stirred for about one hour, and the resulting suspension is allowed to stand overnight, followed by centrifugation to obtain a precipitate.

To effect a dialysis step, the precipitate can be dissolved in 500 ml 0.1M ammonium bicarbonate and exhaustively dialyzed against water. After dialysis, the solution is centrifuged at 100,000 x g for about 90 minutes, thereby to obtain a clear supernatant.

For anion exchange chromatography, solid ammonium bicarbonate can be added to the supernatant to provide a 50 mM solution, pH 7.8. The resulting 50 mM supernatant can then be applied to a 6.5 x 40 cm column of DEAE cellulose (Whatman DE-32) which had been previously equilibrated with 50 mM ammonium bicarbonate, pH 7.8.

After the column is washed with the 50 mM ammonium bicarbonate solution, the concentration of ammonium bicarbonate in the buffer is increased stepwise from 100 mM to 200 mM. The column is then eluted with 500 mM NaCl in the presence of 200 mM ammonium bicarbonate. oPL containing fractions are identified, for example, by means of the radioimmunoassay of Kahn, C.R. Acad. Sci. (Paris), Ser. D. 272: 2808-11 (1971), and then are pooled and concentrated to 50 ml by ultrafiltration.

For the optional first gel filtration step, the concentrate can be applied to a 4.2 x 104 cm Sephadex G-100 column (Pharmacia, Uppsala, Sweden) previously equilibrated with 10 mM ammonium acetate, pH 5.0.

With or without the first gel filtration, further purification is effected via cation exchange chromatography. Preferred for this purpose is a cation exchange column of "Mono-S" (Pharmacia), a sulfonic acid-based resin, equilibrated with 50 mM HEPES buffer (pH 7.8). The column is eluted with an NaCl gradient, 0 to 1.0 M, with ovine placental lactogen activity eluting at around 0.08 M NaCl.

Active fractions are pooled and concentrated, as described above, and desalted on a G-50 Sephadex (superfine) column. This gel filtration -- the second, if a first filtration was effected -- can be carried out by applying the concentrate to a 1.4 x 94 cm Sephadex G-100 column equilibrated with 0.1 M ammonium bicarbonate, pH 8.7. The fractions determined, via a suitable assay, to contain oPL are pooled, concentrated to a volume of 1.5 ml, and lyophilized.

The oPL thus obtained shows a single band when run on a SDS polyacrylamide gel, with an estimated molecular weight of about 21,500. The isoelectric point (pI) of the oPL can be determined by isoelectric focussing on a polyacrylamide gel, resulting in a cluster of bands with pI ranging between about 8.55 and 8.87.

PROBE DESIGN:

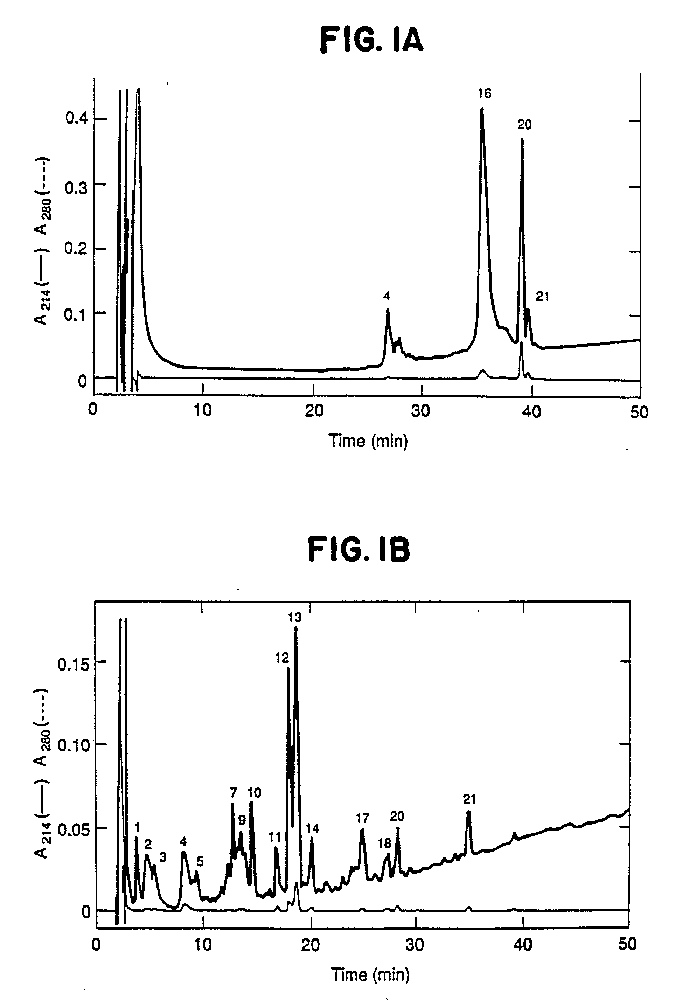

In contrast to nearly all proteins known to bear any structural relation to GH, a sequenceable oPL preparation obtained in the above-described manner was found to be blocked at the N-terminus. As previously described, the problem presented by this blockage was overcome by cleaving the preparation with cyanogen bromide or lysine-c endopeptidase, and separating the peptide fragments by RP-HPLC (see Figure 1).

More specifically, 10 μg of the protein preparation were treated with cyanogen bromide, pursuant to Gross, Methods Enzymol. 11: 238-55 (1967). An additional 10 μg aliquot in 100 mM $NH_4CO_3$ was digested with 0.5 μg of lycine-C endopeptidase (Wako Corp., Dallas, TX) at 37° C for about 24 hours.

The cyanogen bromide-digested sample was mixed with an equal volume of 8 M guanidine hydrochloride, and the resulting mixture was injected directly into a C4 RP-HPLC column (size: 2.1 x 100 mm; SynChrom Inc., Lafayette, IN). In a separate run, the sample digested with lycine-C endopeptidase was injected directly into the same column. For both samples, the column was eluted with a 30 ml, zero-to-70% gradient of 1 propanol in 0.1% TFA. The flow rate was 0.5 ml per minute.

Table 1 shows the sequence that was obtained from each of three peptide peaks isolated by the above-described RP-HPLC. The mixture sequence for peak KC13 was resolved by comparison to the known sequence for ovine prolactin, as well as to the overlapping sequence of peak CN16.

TABLE 1

| Peptide Peak | Sequence | Initial Yield (pmol) |
|---|---|---|
| CN16 | VNRFDEQYGQGINSESKVINXHT | 50 |
| CN20 | VNRFDEQYGQGINXE | 2 |
| KC13 a | LAGEMVNRFDE | 100 |
| KC13 b | NEPYPXWXEQ | 35 |

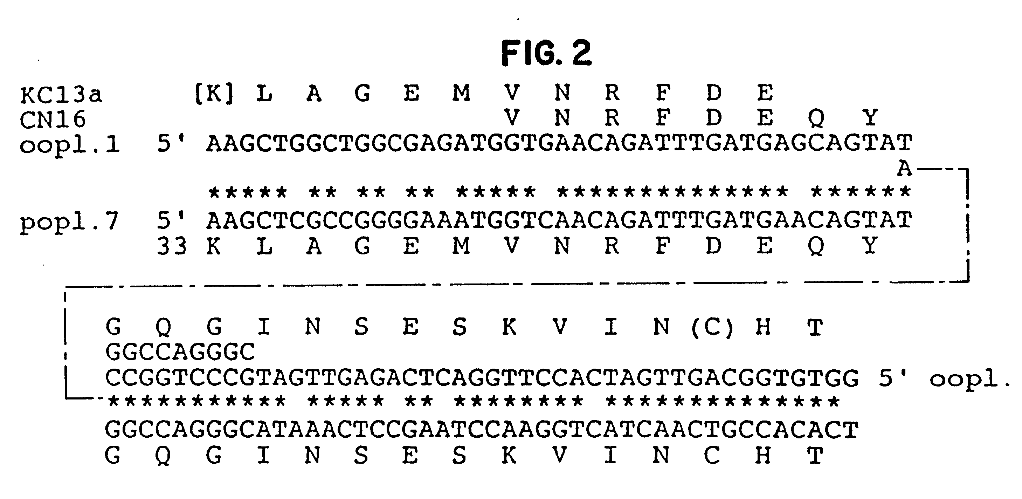

An oligonucleotide probe was chemically synthesized, pursuant to the methodology of Froehler et al., Nucleic Acids Res. 14: 5399-407 (1986), based on a 29 amino-acid sequence determined from peaks KC13a and CN16, which in turn were derived from the sheep placental lactogen protein purified as above. As shown in Figure 2 this probe actually continued two oligonucleotides, oopl.1 (a 51-mer) and oopl.2 (a 46-mer), with a single codon choice for each amino acid. A lysine residue was inferred to be at the amino terminus of the lysine-C peptide KC13a, and a cysteine was used for the uncertain amino-acid at residue 27. The two oligonucleotides were synthesized with a 10 base-pair overlap and labelled by filling in the mixture with labelled nucleotides using the large fragment of DNA polymerase.

A library of 300,000 cDNA clones in the vector λgt10 was generated from 5 μg of ovine placental poly-A + RNA. In accordance with Wood (1987), incorporated by reference above, the library was screened in duplicate on six 150 mm plates with a probe. Hybridization was performed in 5 x SSC, 20% formamide, at 42° C. Filters were washed with 0.5 x SSC at 42° C.

Thirty-two duplicate positives were found, and 1 kbp inserts from two independent clones, λopl.7 and λopl.8, were subcloned into pUC119, see Vieira et al., Methods Enzymol. 152: 3-11 (1987), and sequenced via the method of Sanger et al, Proc. Nat'l Acad. Sci. (USA) 74: 5463-67 (1977). The determined DNA sequence matched well with the probe sequence (Figure 2), with one stretch of 17 and two stretches of 14 nucleotides matching exactly.

Figure 3 gives the full DNA insert sequence from clone λopl.7. Clone λopl.8 begins with nucleotide 8 and ends with nucleotide 908. The DNA sequence of these two clones matches exactly except for nucleotide 9, which is a cytosine (C) in λopl.8. The cDNA sequence contains a single long open reading frame of 236 amino-acids beginning with a methionine residue.

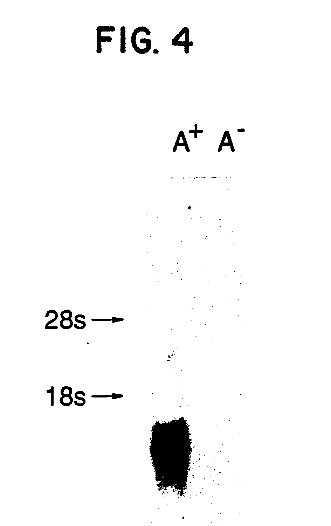

The amino-acid sequences determined from the purified protein (see Table 1) match exactly the amino-acid sequence deduced from the cDNA clones with the exception of a few residues (Figure 3). The putative initiating ATG is the first methionine codon in the 5′ untranslated region of the cDNA clones. The cDNA sequence contains a 3′ untranslated region of about 200 bp. Clone λopl.7 has a 3′ poly-A sequence of 16 bases; 23 bases preceding this is a poly-A addition sequence, AATAAAAAA. See Proudfoot et al., Nature 252: 359-62 (1974). Blot hybridization of ovine placenta RNA with the full-length cDNA insert from clone λopl.7 showed a single, broad band of about 1 kbp (Figure 4), confirming the size of the RNA encoding this hGH-receptor-binding protein.

Mass-spectral analysis was carried out, via molecular ion mass spectroscopy as described, for example, by Biemann and Scoble, Science 237: 992-37 (1987), to determine the mass ion molecular weight of a number of the cyanogen bromide peptides (Figure 1). A major mass peak of 4212.2 was measured for peptide CN4.

The mass thus measured is in close agreement with the predicted mass of 4212.7 for the N-terminal cyanogen bromide fragment beginning with the third possible glutamine residue, which is represented at position +1 (Figure 3). These results indicate that this hGH-receptor-binding protein has a signal sequence of 38 amino-acids and a mature protein beginning with the glutamine shown at position +1 in Figure 3.

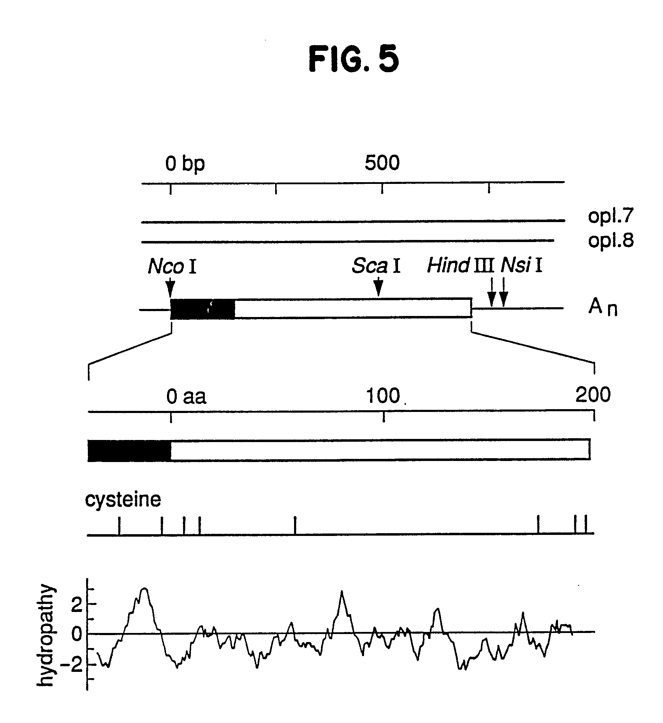

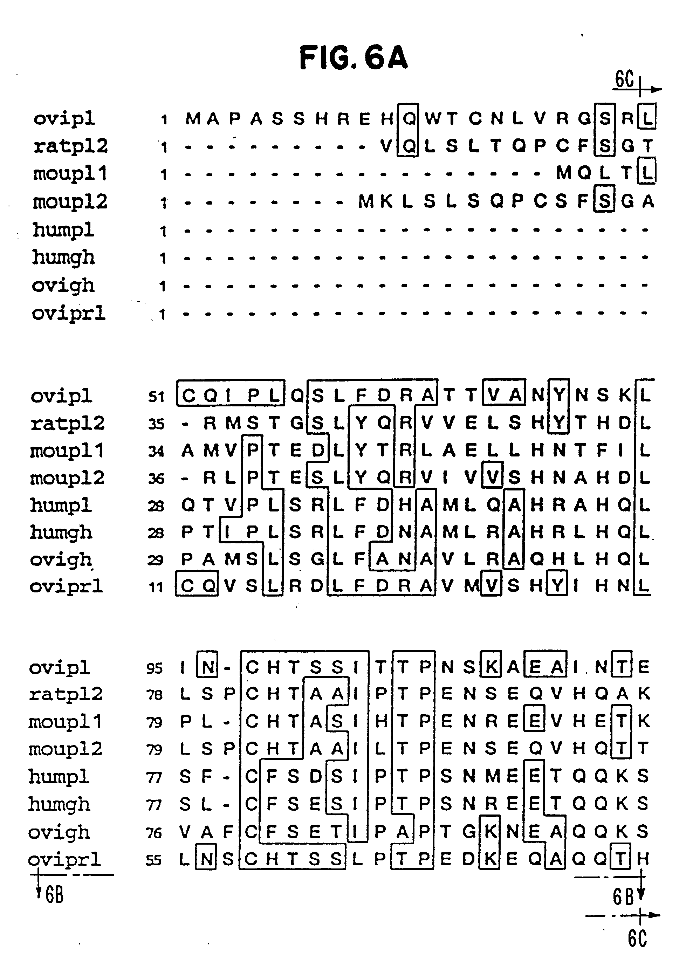

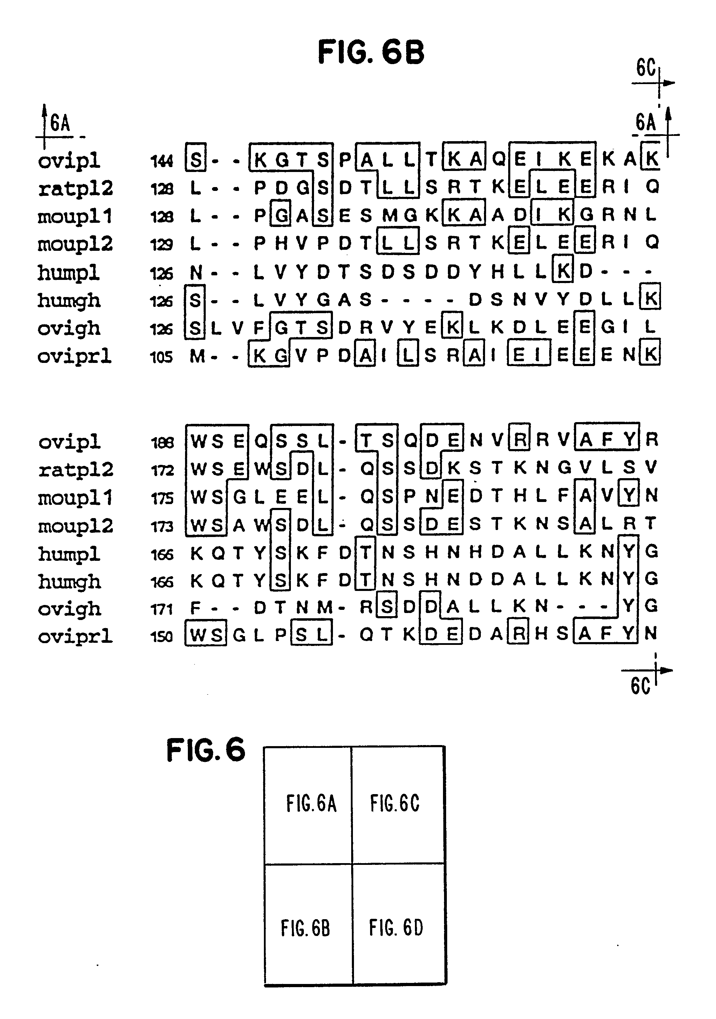

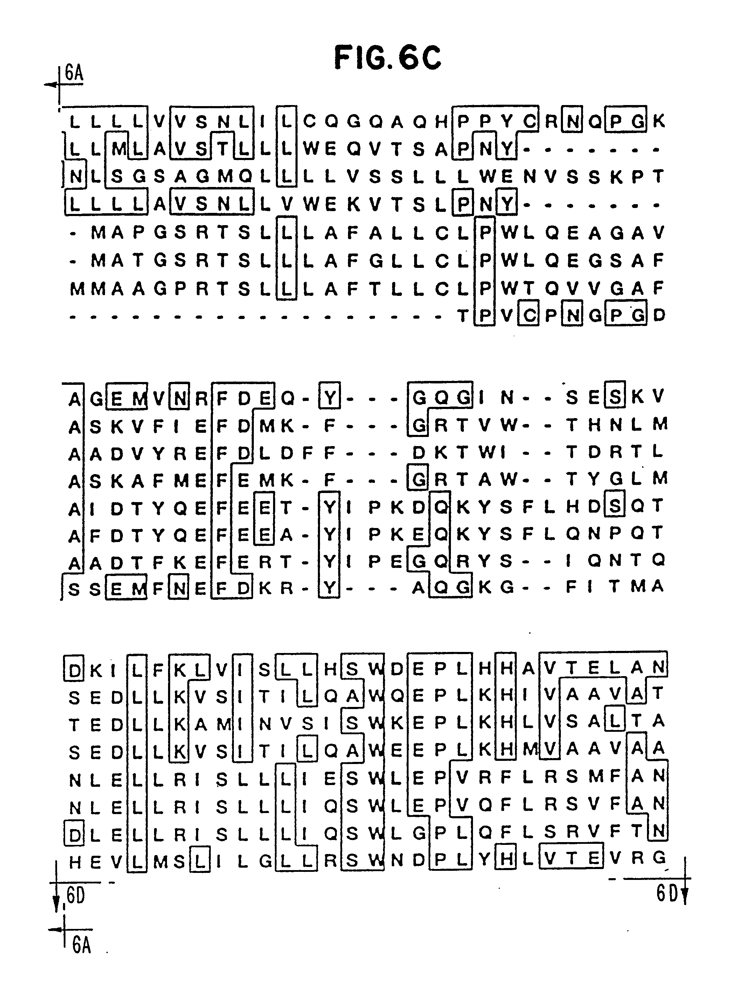

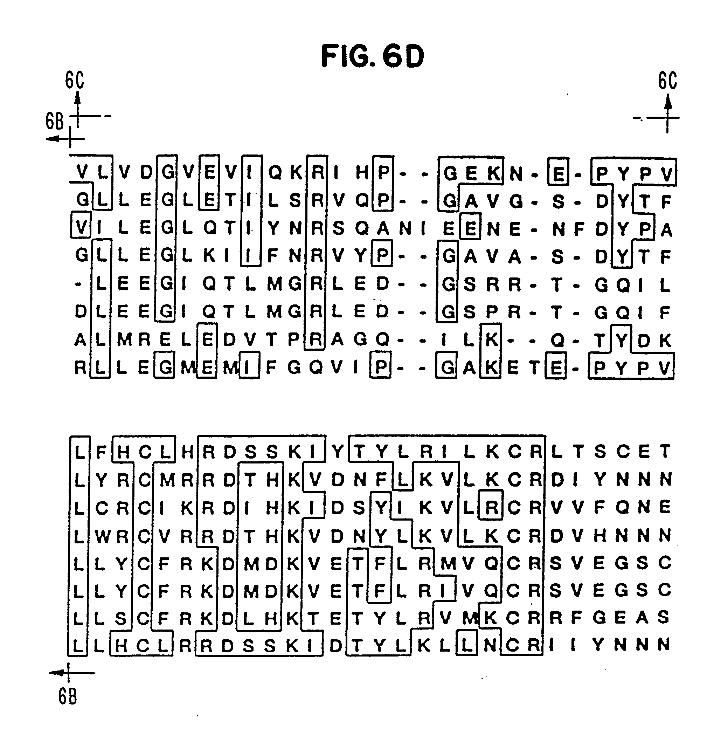

The cDNA sequence predicts a full-length mature protein of 198 amino-acids containing six cysteine residues (Figure 5). Pursuant to the criteria of Marshall, Rev. Biochem. 41: 673-702 (1972), there are no potential N-linked glycosylation sites evident. The hydrophobicity plot, see Kyte & Doolittle, J. Molec. Biol. 157: 105-32 (1982), indicates the presence of a single signal sequence. An alignment of the amino-acid sequence of the mature protein with the sequence of placental lactogens from a number of other species, as well as hGH, ovine growth hormone, and ovine prolactin, shows that this hGH-receptor-binding protein is most similar to the animal placental lactogens (Figure 6). It is remarkable that, although it has only 26% identity of residues with hGH, the aforementioned protein binds hGH receptor as well as hGH itself. This is in contrast to human placental lactogen, which is 85% identical to hGH but does not bind the human GH receptor at all.

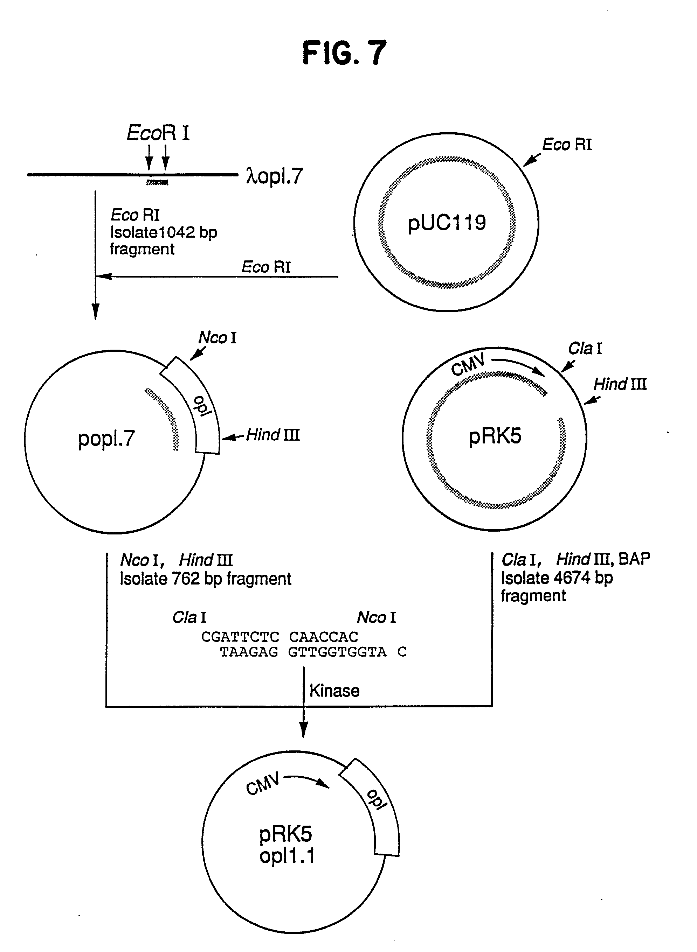

Expression of the hGH-receptor-binding protein can be achieved by cloning the complete open reading frame for the protein in a mammalian expression vector. Figure 7 shows the construction of such a vector, pRK5.opll.1, to express the protein. First, the 1042 bp EcoRI fragment containing the full cDNA insert is isolated from the primary cDNA clone, λopl.7. This fragment is cloned into the vector pUC119, e.g., see Viera et al., supra. which is cut with EcoRI and subjected to the action of a phosphatase to generate plasmid pop1.7. Second, the final plasmid is constructed from three pieces of DNA: (1) a 762 bp NcoI/HindIII fragment of pop1.7, containing the coding sequence of the protein; (2) an approximately 16 bp ClaI/NcoI synthetic, double-strand oligonucleotide that is phosphorylated via the action of a kinase; and (3) a 4674 bp CLaI/HindIII fragment of pRK5, see Suva, et al., Science 237:893-6 (1987), incorporated by reference above, which has been phosphatased. These three fragments are ligated together to generate the final plasmid pRK5.opll.1.

This plasmid can then be transfected into a suitable mammalian tissue culture cell line, such as monkey kidney cell line COS-7 or human kidney cell line 293. The transfection can be performed by the calcium phosphate method disclosed, for example, by Wigler et al., Proc. Nat'l Acad. Sci. (USA) 76: 137376 (1979). At one, two and three days after transfection, the culture medium can be assayed for the hGH-receptor-binding protein by performing a binding assay with a suitable source of GH receptor. This binding is measured as the displacement of $^{125}$I-labelled hGH by culture supernatants containing the expressed protein.

Example 2. Bacterial expression system.

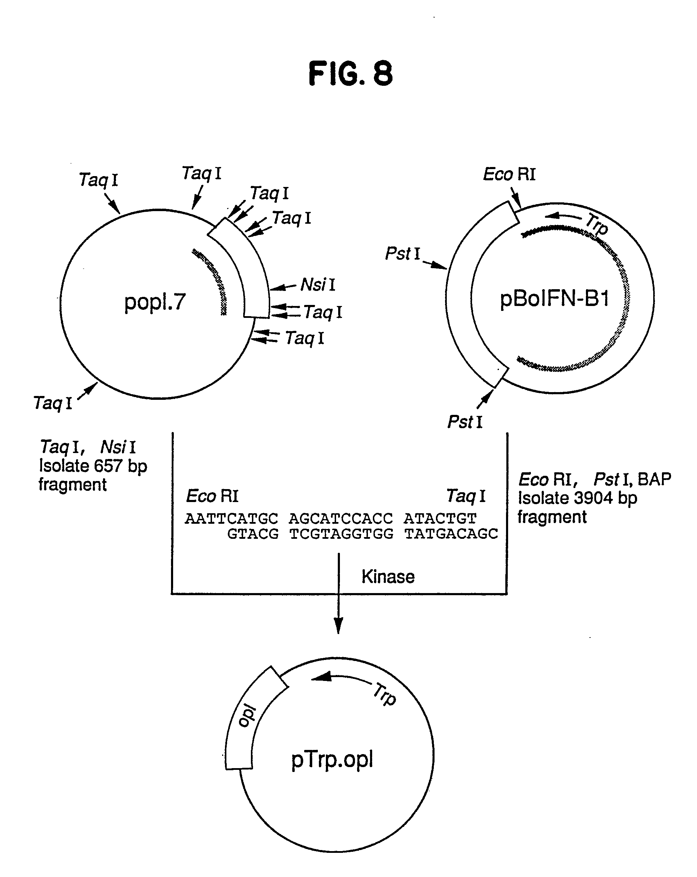

GH-receptor-binding protein as described above can also be obtained via direct expression in E. coli. among other bacterial hosts, of DNA within the present invention. For this purpose, an expression vector containing the tryptophan operon promoter, see Hallewell & Emtage, Gene 9: 27 (1980), is preferably utilized. Figure 8 shows the construction of such a vector, pTrp.opl, that can be used to express the Example 1 protein, preceded by a methionine residue. This plasmid is constructed from three DNA segments: (1) a 657-bp TaqI/NsiI fragment of popl.7 (see Figure 7) which contains most of the coding sequence of the protein; (2) a synthetic, double-stranded oligonucleotide, approximately 25 bp in length, that is phosphorylated via the action of a kinase; and (3) a 3904-bp EcoRI/PstI fragment of plasmid pBoIFN-B1, as described by Leung et al, id. (1984), which has been phosphatased.

These three segments are ligated together to generate the final plasmid pTrp.opl, which can then be transfected into E. coli. The cultured bacterial cells containing the plasmid can be ruptured, for example, by sonication, and the cell extract subjected to a binding assay, as previously described, to ascertain the presence of a GH-receptor-binding protein.


**Claims**

1. An isolated nucleic-acid sequence encoding a protein that binds a mammalian growth hormone receptor, wherein said protein is characterized by an amino-acid-sequence identity of about 26% relative to human growth hormone and about 67% relative to ovine placental lactogen.

2. A nucleic-acid sequence according to claim 1, wherein said growth hormone receptor is an ovine growth hormone receptor.

3. A nucleic-acid sequence according to claim 1, wherein said growth hormone receptor is a human growth hormone receptor.

4. A nucleic-acid sequence according to claim 1, wherein said protein encoded by said nucleotide sequence comprises an amino-acid sequence represented as follows:

Q-H-P-P-Y-C-R-N-Q-P-G-K-C-Q-I-P-L-Q-S-L-F-D-R-A-T-T-V-A-N-Y-N-S-K-L-A-G-E-M-V-N-R-F-D-E-Q-Y-G-Q-
G-I-N-S-E-S-K-V-I-N-C-H-T-S-S-I-T-T-P-N-S-K-A-E-A-I-N-T-E-D-K-I-L-F-K-L-V-I-S-L-L-H-S-W-D-E-P-L-H-H-A-
V-T-E-L-A-N-S-K-G-T-S-P-A-L-L-T-K-A-Q-E-I-K-E-K-A-K-V-L-V-D-G-V-E-V-I-Q-K-R-I-H-P-G-E-K-N-E-P-Y-P-V-
W-S-E-Q-S-S-L-T-S-Q-D-E-N-V-R-R-V-A-F-Y-R-L-F-H-C-L-H-R-D-S-S-K-I-Y-T-Y-L-R-I-L-K-C-R-L-T-S-C-E-T.

5. A nucleic-acid sequence according to claim 1, wherein said nucleotide sequence comprises a sequence represented as follows:

```
5'  CAGCAT CCACCATACT GTCGAAACCA ACCTGGAAAG
3'  GTCGTA GGTGGTATGA CAGCTTTGGT TGGACCTTTC

TGCCAGATTC CCCTTCAAAG CCTGTTTGAC AGAGCAACAA
ACGGTCTAAG GGGAAGTTTC GGACAAACTG TCTCGTTGTT

CGGTGGCTAA CTACAACTCT AAGCTCGCCG GGGAAATGGT
GCCACCGATT GATGTTCAGA TTCGAGCGGC CCCTTTACCA

CAACAGATTT GATGAACAGT ATGGCCAGGG CATAAACTCC
GTTGTCTAAA CTACTTGTCA TACCGGTCCC GTATTTGAGG

GAATCCAAGG TCATCAACTG CCACACTTCA TCCATCACCA
CTTAGGTTCC AGTAGTTGAC GGTGTGAAGT AGGTAGTGGT

CCCCTAATAG CAAAGCAGAA GCTATAAATA CAGAGGACAA
GGGGATTATC GTTTCGTCTT CGATATTTAT GTCTCCTGTT

AATCCTGTTT AAGTTGGTTA TCAGTTTGCT ACACTCATGG
TTAGGACAAA TTCAACCAAT AGTCAAACGA TGTGAGTACC

GATGAACCTC TGCATCATGC AGTCACAGAG CTGGCAAACT
CTACTTGGAG ACGTAGTACG TCAGTGTCTC GACCGTTTGA

CAAAAGGAAC CTCACCTGCT CTCTTGACAA AGGCTCAAGA
GTTTTCCTTG GAGTGGACGA GAGAACTGTT TCCGAGTTCT

GATTAAGGAA AAGGCCAAAG TACTTGTAGA CGGTGTGGAA
CTAATTCCTT TTCCGGTTTC ATGAACATCT GCCACACCTT

GTGATACAAA AAAGGATTCA TCCTGGAGAG AAGAACGAGC
CACTATGTTT TTTCCTAACT AGGACCTCTC TTCTTGCTCG

CCTATCCAGT GTGGTCAGAA CAGTCCTCCC TGACATCACA
GGATAGGTCA CACCAGTCTT GTCAGGAGGG ACTGTAGTGT

GGATGAGAAT GTGCGCCGAG TTGCTTTTTA TAGACTGTTC
CCTACTCTTA CACGCGGCTC AACGAAAAAT ATCTGACAAG

CACTGCCTAC ACAGGGATTC AAGTAAAATT TACACCTACC
GTCACGGATG TGTCCCTAAG TTCATTTTAA ATGTGGATGG

TCCGGATACT TAAGTGCCGA TTGACCTCAT GCGAAACC   3'
AGGCCTATGA ATTCACGGCT AACTGGAGTA CGCTTTGG   5'
```

6. A nucleic-acid sequence according to claim 1, wherein said sequence hybridizes to an oligonucleotide probe, said probe comprising a nucleotide sequence represented as follows:

5′

AGCTCGCCGGGGAAATGGTCAACAGATTTGATGAACAGTATGGCCAG-

TCGAGCGGCCCCTTTACCAGTTCTGTAAACTACTTGTCATACCGGTC-

3′

3′

GGCATAAACTCCGAATCCAAGGTCATCAACTGCCACACT

CCGTATTTGAGGCTTAGGTTCCAGTAGTTGACGGTGTGA ,

5′

when the hybridizing occurs at 42° C in 5 x SSC, 20% formamide, and is followed by washing with 0.5 x SSC at 37° C.

7. A vector containing a nuclei-acid sequence according to any one of claims 1 to 6.

8. A process of preparing a DNA molecule comprising preparing cDNA from mRNA extracted from placental cotyledons and isolating from the cDNA a DNA sequence comprised of a nucleic-acid sequence according to any one of claims 1 to 6.

9. A process of preparing a DNA molecule comprising preparing a cDNA library from mRNA extracted from placental cotyledons, probing the library with a hybridisation probe selected from the probes shown in Figure 2, sequencing the cDNA from strongly hybridising colonies and using the cDNA to obtain a DNA sequence comprised of a nucleic-acid sequence according to any one of claims 1 to 6.

10. A transformed cell line comprising a nucleic-acid sequence according to any one of claims 1 to 6 or prepared according to the process of claim 8 or claim 9 or comprising a vector according to claim 7.

11. A composition comprising (a) a homogeneous protein that binds a mammalian growth hormone receptor and that is characterized by an amino-acid sequence identity of about 26% relative to human growth hormone and about 67% relative to bovine placental lactogen, and (b) a physiologically-compatible carrier therefor.

12. A composition according to claim 11, wherein said homogeneous protein is present in pure form and consists essentially of an amino-acid sequence of residue +1 (gln) through residue +198 (thr) in Figure 3.

13. A homogeneous protein that binds a mammalian growth hormone receptor and that is characterised by an amino-acid-sequence identity of about 26% relative to human growth hormone and about 67% relative to bovine placental lactogen, or a composition according to claim 11 or claim 12, for use in stimulating milk production in a mammal.

14. A homogeneous protein consisting essentially of an amino-acid sequence of residue +1 (gln) through residue +198 (thr) in Figure 3 for use in stimulating milk production in a mammal.

# FIG. IA

# FIG. IB

# FIG. 2

```
KC13a        [K] L   A   G   E   M   V   N   R   F   D   E
CN16                                 V   N   R   F   D   E   Q   Y
oopl.1    5' AAGCTGGCTGGCGAGATGGTGAACAGATTTGATGAGCAGTAT
                                                                 A———┐
          ***** ** ** ** ***** ****************** ******        ¦
popl.7    5' AAGCTCGCCGGGGAAATGGTCAACAGATTTGATGAACAGTAT          ¦
          33 K   L   A   G   E   M   V   N   R   F   D   E   Q   Y
```

```
          G   Q   G   I   N   S   E   S   K   V   I   N  (C)  H   T
          GGCCAGGGC
          CCGGTCCCGTAGTTGAGACTCAGGTTCCACTAGTTGACGGTGTGG 5' oopl.
          *********** ***** ** ******** ***************
          GGCCAGGGCATAAACTCCGAATCCAAGGTCATCAACTGCCACACT
          G   Q   G   I   N   S   E   S   K   V   I   N   C   H   T
```

# FIG. 3A

3B→
|
S

-68 TTTTTTTT TTTTTGGGC CATCTCCCCA TCAGCAGCTG TCCTCATC
-38

 31 CAATGGACTT GCAACCTTGT TCGAGGGTCC CGCCTGCTTC TGCTGCTG
-28 Q   W   T   C   N   L   V   R   G   S   R   L   L   L   L

131 GTCGAAACCA ACCTGGAAAG TGCCAGATTC CCCTTCAAAG CCTGTTTG
  7 R   N   Q   P   G   K   C   Q   I   P   L   Q   S   L   F   D

                                                    STYI
231 CAACAGATTT GATGAACAGT ATGGCCAGGG CATAAACTCC GAATCCAA
 40 N   R   F   D   E   Q   Y   G   Q   G   I   N   S   E   S   K
                                                            C
                              CN20               •

331 GCTATAAATA CAGAGGACAA AATCCTGTTT AAGTTGGTTA TCAGTTTG
 73 A   I   N   T   E   D   K   I   L   F   K   L   V   I   S   L

431 CAAAAGGAAC CTCACCTGCT CTCTTGACAA AGGCTCAAGA GATTAAGG
107 K   G   T   S   P   A   L   L   T   K   A   Q   E   I   K   E

531 TCCTGGAGAG AAGAACGAGC CCTATCCAGT GTGGTCAGAA CAGTCCTC
140 P   G   E   K   N   E   P   Y   P   V   W   S   E   Q   S   S
                        KC13B          •          •

631 CACTGCCTAC ACAGGGATTC AAGTAAAATT TACACCTACC TCCGGATA
173 H   C   L   H   R   D   S   S   K   I   Y   T   Y   L   R   I

                                        HINDIII
731 GAGATGGTTA GTGATGATCC ATCCTGTCAA AAGCTTCTTT GAGTTTTA

831 TTGTAGAGAT GTCAAAATCT AAGAAGGAAA TTTGTCAATG TCTTTATC
                                                    3B→

# FIG. 3B

```
|—3A
|                              STYI
|                              NCOI
TYI
CT AGGATTTCTC TCCAATCCCC ATGGCTCCAG CATCCAGCCA CCGTGAGCAC
                          M  A  P  A   S  S  H   R  E  H


GT GGTATCAAAT CTAATCTTGT GCCAGGGTCA GGCACAGCAT CCACCATACT
V  V  S  N   L  I  L  C   Q  G  Q   A  Q  H   P  P  Y  C


AC AGAGCAACAA CGGTGGCTAA CTACAACTCT AAGCTCGCCG GGGAAATGGT
   R  A  T  T   V  A  N   Y  N  S   K  L  A  G   E  M  V
                                                      |—
                                                      |—
                                    |—    KC13A    |—


GG TCATCAACTG CCACACTTCA TCCATCACCA CCCCTAATAG CAAAGCAGAA
   V  I  N  C   H  T  S   S  I  T  T   P  N  S   K  A  E
   |—   •   —|
   N16


CT ACACTCATGG GATGAACCTC TGCATCATGC AGTCACAGAG CTGGCAAACT
L  H  S  W   D  E  P  L   H  H  A   V  T  E   L  A  N  S


                SCAI
AA AAGGCCAAAG TACTTGTAGA CGGTGTGGAA GTGATACAAA AAAGGATTCA
   K  A  K  V   L  V  D   G  V  E   V  I  Q  K   R  I  H


CC TGACATCACA GGATGAGAAT GTGCGCCGAG TTGCTTTTTA TAGACTGTTC
   L  T  S  Q   D  E  N   V  R  R  V   A  F  Y   R  L  F


CT TAAGTGCCGA TTGACCTCAT GCGAAACCTA GAATTAACCC AGCCAATCCT
L  K  C  R   L  T  S  C   E  T  O


                NSII
TA GCTATTTAAT GCATGTTTGG GTCTTATCTG AAACAAAATA AGCACAGATT


TT CATTTAATAA AAAATCTAAA GAAAATCCAA AAAAAAAAAA AAAA
|
|—3A
```

# FIG. 4

$A^+$ $A^-$

28s →

18s →

# FIG. 5

# FIG. 6A

6C →

```
ovipl    1  M A P A S S H R E H Q W T C N L V R G S R L
ratpl2   1  - - - - - - - - - - V Q L S L T Q P C F S G T
moupl1   1  - - - - - - - - - - - - - - - M Q L T L
moupl2   1  - - - - - - M K L S L S Q P C S F S G A
humpl    1  - - - - - - - - - - - - - - - - - - - -
humgh    1  - - - - - - - - - - - - - - - - - - - -
ovigh    1  - - - - - - - - - - - - - - - - - - - -
oviprl   1  - - - - - - - - - - - - - - - - - - - -
```

```
ovipl   51  C Q I P L Q S L F D R A T T V A N Y N S K L
ratpl2  35  - R M S T G S L Y Q R V V E L S H Y T H D L
moupl1  34  A M V P T E D L Y T R L A E L L H N T F I L
moupl2  36  - R L P T E S L Y Q R V I V S H N A H D L
humpl   28  Q T V P L S R L F D H A M L Q A H R A H Q L
humgh   28  P T I P L S R L F D N A M L R A H R L H Q L
ovigh   29  P A M S L S G L F A N A V L R A Q H L H Q L
oviprl  11  C Q V S L R D L F D R A V M V S H Y I H N L
```

```
ovipl   95  I N - C H T S S I T T P N S K A E A I N T E
ratpl2  78  L S P C H T A A I P T P E N S E Q V H Q A K
moupl1  79  P L - C H T A S I H T P E N R E E V H E T K
moupl2  79  L S P C H T A A I L T P E N S E Q V H Q T T
humpl   77  S F - C F S D S I P T P S N M E E T Q Q K S
humgh   77  S L - C F S E S I P T P S N R E E T Q Q K S
ovigh   76  V A F C F S E T I P A P T G K N E A Q Q K S
oviprl  55  L N S C H T S S L P T P E D K E Q A Q Q T H
```

↓6B                                                    6B↓

6C

# FIG. 6B

```
        6C

       6A'

6A
ovipl   144  S - - K G T S P A L L T K A Q E I K E K A K
ratpl2  128  L - - P D G S D T L L S R T K E L E E R I Q
moupl1  128  L - - P G A S E S M G K K A A D I K G R N L
moupl2  129  L - - P H V P D T L L S R T K E L E E R I Q
humpl   126  N - - L V Y D T S D S D D Y H L L K D - - -
humgh   126  S - - L V Y G A S - - - - D S N V Y D L L K
ovigh   126  S L V F G T S D R V Y E K L K D L E E G I L
oviprl  105  M - - K G V P D A I L S R A I E I E E E N K


ovipl   188  W S E Q S S L - T S Q D E N V R R V A F Y R
ratpl2  172  W S E W S D L - Q S S D K S T K N G V L S V
moupl1  175  W S G L E E L - Q S P N E D T H L F A V Y N
moupl2  173  W S A W S D L - Q S S D E S T K N S A L R T
humpl   166  K Q T Y S K F D T N S H N H D A L L K N Y G
humgh   166  K Q T Y S K F D T N S H N D D A L L K N Y G
ovigh   171  F - - D T N M - R S D D A L L K N - - - Y G
oviprl  150  W S G L P S L - Q T K D E D A R H S A F Y N

                                                6C
```

# FIG. 6

|  |  |
|---|---|
| FIG. 6A | FIG. 6C |
| FIG. 6B | FIG. 6D |

# FIG. 6C

```
6A

L L L L V V S N L I L C Q G Q A Q H P P Y C R N Q P G K
L L M L A V S T L L L W E Q V T S A P N Y - - - - - - -
N L S G S A G M Q L L L L V S S L L L W E N V S S K P T
L L L L A V S N L L V W E K V T S L P N Y - - - - - - -
- M A P G S R T S L L L A F A L L C L P W L Q E A G A V
- M A T G S R T S L L L A F G L L C L P W L Q E G S A F
M M A A G P R T S L L L A F T L L C L P W T Q V V G A F
- - - - - - - - - - - - - - - - - - - T P V C P N G P G D


A G E M V N R F D E Q - Y - - - G Q G I N - - S E S K V
A S K V F I E F D M K - F - - - G R T V W - - T H N L M
A A D V Y R E F D L D F F - - - D K T W I - - T D R T L
A S K A F M E F E M K - F - - - G R T A W - - T Y G L M
A I D T Y Q E F E E T - Y I P K D Q K Y S F L H D S Q T
A F D T Y Q E F E E A - Y I P K E Q K Y S F L Q N P Q T
A A D T F K E F E R T - Y I P E G Q R Y S - - I Q N T Q
S S E M F N E F D K R - Y - - - A Q G K G - - F I T M A


D K I L F K L V I S L L H S W D E P L H H A V T E L A N
S E D L L K V S I T I L Q A W Q E P L K H I V A A V A T
T E D L L K A M I N V S I S W K E P L K H L V S A L T A
S E D L L K V S I T I L Q A W E E P L K H M V A A V A A
N L E L L R I S L L L I E S W L E P V R F L R S M F A N
N L E L L R I S L L L I Q S W L E P V Q F L R S V F A N
D L E L L R I S L L L I Q S W L G P L Q F L S R V F T N
H E V L M S L I L G L L R S W N D P L Y H L V T E V R G

6D                                                6D
6A
```

## FIG. 6D

6C      6C

6B

```
V L V D G V E V I Q K R I H P - - G E K N - E - P Y P V
G L L E G L E T I L S R V Q P - - G A V G - S - D Y T F
V I L E G L Q T I Y N R S Q A N I E E N E - N F D Y P A
G L L E G L K I I F N R V Y P - - G A V A - S - D Y T F
- L E E G I Q T L M G R L E D - - G S R R - T - G Q I L
D L E E G I Q T L M G R L E D - - G S P R - T - G Q I F
A L M R E L E D V T P R A G Q - - I L K - - Q - T Y D K
R L L E G M E M I F G Q V I P - - G A K E T E - P Y P V
```

```
L F H C L H R D S S K I Y T Y L R I L K C R L T S C E T
L Y R C M R R D T H K V D N F L K V L K C R D I Y N N N
L C R C I K R D I H K I D S Y I K V L R C R V V F Q N E
L W R C V R R D T H K V D N Y L K V L K C R D V H N N N
L L Y C F R K D M D K V E T F L R M V Q C R S V E G S C
L L Y C F R K D M D K V E T F L R I V Q C R S V E G S C
L L S C F R K D L H K T E T Y L R V M K C R R F G E A S
L L H C L R R D S S K I D T Y L K L L N C R I I Y N N N
```

6B

# FIG. 7

# FIG. 8

*Taq* I     *Taq* I     *Taq* I

*Taq* I

*Nsi* I

*Taq* I

*Taq* I

popl.7

*Taq* I

*Eco* RI

*Trp*

pBoIFN-B1

*Pst* I

*Pst* I

*Taq* I,  *Nsi* I
Isolate 657 bp
fragment

*Eco* RI,  *Pst* I, BAP
Isolate 3904 bp
fragment

*Eco* RI                 *Taq* I

```
AATTCATGC AGCATCCACC ATACTGT
     GTACG TCGTAGGTGG TATGACAGC
```

Kinase

*Trp*

opl

pTrp.opl